# EUROPEAN PATENT APPLICATION

(11) **EP 2 881 104 A1**
(43) Date of publication of application: **10.06.2015**
(21) Application number: 13195821.7
(22) Date of filing: 05.12.2013
(51) Int. Cl.: A61K 8/55, A61Q 15/00

(54) **Use of compositions comprising phosphatidylserine and phosphatidic acid and/or salts thereof for reducing or preventing sweating**

(71) Applicant: Lonza Ltd, 3930 Visp (CH)
(72) Inventor: Freitas, Ulla, 79540 Lörrach (DE); Rüsing, Matthias, 50935 Köln (DE)
(74) Representative: Banse & Steglich

(57) **Abstract**

The invention relates to uses of a composition comprising phosphatidylserine and phosphatidic acid and/or salts thereof for reducing or preventing sweating. Preferably, the composition comprises 5 to 50 wt.% phosphatidylserine and lysophosphatidylserine, 5 to 50 wt.% phosphatidic acid and lysophosphatidic acid and/or salts thereof, and 0 to 90 wt% additional phospholipids and glycerides. The invention also relates to medicaments for reducing or preventing sweating and methods for using such compositions.

## Description

The invention relates to uses of a composition comprising phosphatidylserine and phosphatidic acid and/or salts thereof for reducing or preventing sweating. The invention also relates to medicaments for reducing or preventing sweating and methods for using such compositions.

### Background of the invention

Sweating (transpiration, perspiration) is the production of fluids secreted by the sweating glands in the skin of mammals. Sweating is a normal means of regulation of the human body temperature. However, excessive sweating (hyperhidrosis) can be a severe problem to individuals. Hyperhidrosis is often associated with a decrease of life quality from a psychological, emotional, and social perspective.

Various treatments for excessive sweating are known in the art. A common method is topical application of aluminium chloride. However, the efficiency is limited and treatment is associated with skin irritation.

Botulinum toxine-type A (botox) injections are applied to block sweat glands for several months. However, side effects of Botox are still under debate, and the treatment is relatively expensive, complicated and painful.

Various anticholinergic drugs, such as oxybutynin and glykopyrrolate, are used for treating hyperhidrosis. However, such agents have considerable side effects, because they block the neurotransmitter acetylcholine in the peripheral and central nervous system.

Overall, a need exists for alternative and improved agents for reducing or preventing sweating, which are easily available, and which cause no or only acceptable side effects.

Hellhammer et al., 2004, found that a combination product of phosphatidylserine and phosphatidic acid reduces cortisol levels and enhances psychological state under acute social stress (Hellhammer et al., "Effects of soy lecithin phosphatidic acid and phosphatidylserine complex (PAS) on the endocrine response to mental stress", 2004, Stress, 7(2), 119-126). In the underlying study, a combination product comprising phosphatidylserine and phosphatidic acid (the "PAS complex"; PAS) was administered to individuals for 42 days. The individuals were subjected to a Trier Social Stress Test (TSST). It was found that consumption of "400 mg PAS" daily dampens saliva cortisol, serum cortisol and ACTH levels which are common hormonal markers for stress (according to Hellhammer et al., "100 mg PAS" correspond to 100 mg phosphatidylserine, 125 mg phosphatidic acid and 270 mg other phospholipids). In addition, it was found that "200 mg PAS" daily can decrease perceived stress, measured via questionnaires. However, the heart-rate response to stress was not dampened and it was concluded that the administration had no effect on the hypophtalamic level.

### Problem underlying the invention

The problem underlying the invention is to provide compositions, uses and methods, which overcome the above-mentioned drawbacks. The problem underlying the invention is to provide novel and improved compositions, methods and uses for reducing or preventing sweating. Specifically, the problem is to provide compositions, methods and uses for reducing or preventing hyperhidrosis, hand-sweating and sweating associated with stress. The inventive solution shall be simple, cost-efficient and easily available. Side effects shall be negligible or absent.

### Disclosure of the invention

Surprisingly, the problem underlying the invention is solved by the uses, medicaments and methods according to the claims. Further embodiments of the invention are disclosed throughout the description.

Subject of the invention is the use of a composition comprising phosphatidylserine and phosphatidic acid and/or salts thereof for reducing or preventing sweating. The inventive use can be non-therapeutic or therapeutic.

The inventive use is for reducing or preventing sweating. The term "reducing" as used herein means that sweating is decreased or inhibited.

Typically, the inventive use is for human individuals. Non-human animals often do not produce large amounts of sweat. However, extensive sweating is known from horses and primates.

Preferably, the inventive use is for reducing or preventing sweating of hands, feet, armpits and/or the groin area. Hands, feet, armpits, and the groin area are among the most active regions of sweating due to the relatively high concentration of sweat glands.

In a preferred embodiment of the invention, the sweating is hand sweating. The sweating may be measured on the hand back (dorsal) or on another hand region, such as the palm. However, it has been shown in the art that sweating at specific body regions, such as the hand back, correlates strongly with overall sweating. Therefore, hand sweating is indicative of overall sweating (Allen et al., J. Physiol. 1973, 235, p749-759; Patterson et al., Exp. Physiol. 2000, 85.6, p870-876). Typically, sweating of the hand is measured on the hand back.

Sweating is associated with the increase of moisture on the skin. Sweating can be monitored quantitatively by measuring electrodermal activity on the skin. Electrodermal activity is also referred to as skin conductance or electrodermal response. A high electrical conductance of the skin, to which electrodes are applied, is indicative of sweating. In a preferred embodiment, the inventive use reduces electrodermal activity of the skin by at least 10%, preferably by at least 20% or by at least by 50%, compared to individuals which have not been subjected to the inventive treatment.

In a preferred embodiment of the invention, the sweating, especially the hand sweating, is associated with stress. Stress is an individual reaction to external and internal challenges that ranges from behavioral to molecular adaptions. The hypothalamic-pituitary-adrenal (HPA)-axis plays a major role in stress adaption and releases so-called stress hormones. The HPA-axis is involved in numerous processes such as energy balance, regulation of the immune system and mood. In response to acute stressors, a certain hormonal cascade is activated: The hypothalamus secretes corticotropin-releasing hormone (CRH), which triggers the release of adrenocorticotrophic hormone (ACTH) from the pituitary gland. ACTH then provokes the release of cortisol from the cortex of the suprarenal gland. Cortisol plays a regulatory role in the carbohydrate, lipid and protein metabolism.

The response to acute stress can be measured with the Trier Social Stress Test (TSST; Kirschbaum et al., The 'Trier Social Stress Test'--a tool for investigating psychobiological stress responses in a laboratory setting; Neuropsychobiology 28; 76-81). With the TSST as a stressor, it can be determined if, and to what extent, sweating, such as hand sweating, is reduced in individuals, which have consumed the composition according to the invention, in comparison to individuals, who have consumed a placebo.

In a preferred embodiment of the invention, the sweating is associated with hyperhidrosis. In this embodiment, an individual suffers from hyperhidrosis, and the sweating is a result of the hyperhidrosis. Hyperhidrosis is characterized by abnormally increased sweating, in excess of that required for regulation of body temperature.

The hyperhidrosis may be a generalized or secondary hyperhidrosis. Generalized or secondary hyperhidrosis usually involves the body as a whole and is the result of an underlying condition.

In another embodiment of the invention, the hyperhidrosis is a primary or focal hyperhidrosis. When excessive sweating is localized to certain body areas, such as hands or feet, it is referred to as primary or focal hyperhidrosis. Unlike generalized or secondary hyperhidrosis, it is idiopathic or not associated with an underlying condition.

In a preferred embodiment, the inventive use is for hand sweating associated with hyperhidrosis *(Hyperhidrosis palmiaris).*

In a preferred embodiment, the sweating associated with stress is a result of a generalized or secondary hyperhidrosis, in which the underlying condition is stress.

The composition used according to the invention comprises phosphatidylserine. Phosphatidylserine (phosphatidyl-L-serine, PS, CAS number 8002-43-5) is a phospholipid component, usually kept on the inner-side (the cytosolic side) of cell membranes by the enzyme flippase. It is presumed in the art that administration of phosphatidylserine may dampen cortisol responses to acute exercise and mental stress.

The composition used according to the invention comprises phosphatidic acid (PA) and/or salts thereof. Phosphatidic acid is the acid form of phosphatidate and part of common phospholipids. Phosphatidic acid consists of a glycerol backbone having two hydroxyl groups esterified with fatty acids, and a phosphate group attached to the third hydroxyl group. It is a precursor for other lipids such as phosphatidylserine (PS) or phosphatidylcholine. Phosphatidic acid influences membrane curvature and acts as a signalling lipid.

The composition used according to the invention comprises phosphatidylserine and phosphatidic acid and/or salts thereof. A combination of phosphatidylserine and phosphatidic acid is also referred to as "phosphatidylserine and phosphatidic acid complex" in the prior art. Without being bound to theory, the phospholipids may interact and form complex-like structures. The interactions may resemble those in membranes.

The present application relates to phospholipid compositions. Phospholipids often comprise charged moieties or acidic moieties. According to the invention, the acidic phospholipids, such as phosphatidic acid and lysophosphatidic acid, may be in the acid form, or neutralized, or partially neutralized. Preferably, the phospatidic acid and lysophosphatidic acid is in the acid form. Typically, the composition comprises cations, such as calcium.

In a preferred embodiment of the invention, the composition comprises
(a) phosphatidylserine and lysophosphatidylserine, and
(b) phosphatidic acid and lysophosphatidic acid and/or salts thereof.
   Lysophosphatidylserine and lysophosphatidic acid are derivatives of phosphatidylserine and phosphatidic acid with only one fatty acid residue. The glycerol backbone comprises a free hydroxyl group.
   In a preferred embodiment of the invention, the ratio of component (a) to component (b) is between 5:1 and 1:2, preferably between 2:1 and 1:2, more preferably between 1.5:1 and 1:1.5. Most preferably, the ratio is about 1:1.
   In a preferred embodiment of the invention, the amount of component (a) and/or of component (b) in the composition is at least 5 wt.% each, preferably at least 10 wt.% each, more preferably at least 15 wt.% each, based on the total amount of lipids in the composition. Preferably, the amount is between 5 to 50 wt.% each, preferably between 15 to 40 wt.% each, based on the total amount of lipids in the composition.
   In a preferred embodiment of the invention, the composition comprises
(c) additional phospholipids and glycerides.
   In this respect, the term "additional" means that component (c) does not comprise phospholipids as in component (a) and (b). The additional lipids (c) are preferably inert. This means, that they do not affect sweating.

Preferably, the amounts of components (a), (b) and (c) add up to at least 50 wt.%, preferably at least 75 or at least 90 wt.% of the composition. Preferably, components (a), (b) and (c) are the total of phospholipids and glycerols in the composition. The composition may comprise additional carriers and/or excipients. In a preferred embodiment, the composition does not comprise other pharmacologically active ingredients besides components (a), (b) and (c).

However, also within the scope of the invention is a composition comprising at least one other additional pharmacologically active ingredient. In a preferred embodiment, the composition comprises at least one additional active agent. Preferably, the additional active agent also reduces or prevents sweating, and preferably is active against hyperhidrosis.

In a preferred embodiment, the additional active agent is an anticholinergic agent. Systemic agents used to treat hyperhidrosis include anticholinergic medications. Anticholinergics, such as propantheline bromide, glycopyrrolate, oxybutynin and benztropine are effective because the preglandular neurotransmitter for sweat secretion is acetylcholine (although the sympathetic nervous system innervates the eccrine sweat glands).

In addition, other systemic medications, such as sedatives and tranquilizers, indomethacin, and calcium channel blockers, which may be beneficial in the treatment of palmoplantar hyperhidrosis, can be used as additional active agents.

In another preferred embodiment, the additional active agent is a phytotherapeutic agent. An example is sage. Sage *(Salvia officinalis)* contains phytoestrogen substances that are effectively used to treat hot flashes and to decrease perspiration in both daytime and night-time excessive sweating.

In a preferred embodiment of the invention, the composition comprises
(a) 5 to 50 wt.%, preferably 15 to 40 wt.%, phosphatidylserine and lysophosphatidylserine,
(b) 5 to 50 wt.%, preferably 15 to 40 wt.%, phosphatidic acid and lysophosphatidic acid and/or salts thereof, and
(c) 0 to 90 wt%, preferably 25 to 75 wt.%, additional phospholipids and glycerides.

Preferably, components (a), (b) and (c) add up to 100%. The composition may comprise additional lipids, such as sterols. Preferably, the amount of additional lipids is low. Preferably, components (a), (b) and (c) add up to more than 80%, more than 90% or more than 95% (w/w), or about 100% of the total amounts of lipids in the composition. Preferably, the composition consists of components (a), (b) and (c), optionally with carriers and/or excipients. Preferably, the composition comprises at least 50 wt.%, more preferably at least 75 wt.%, or at least 90 wt.%, most preferably at least 95 wt.% total phospholipids and glycerides (a), (b) and (c).

Preferably, the composition is derived from a natural product. Especially in this embodiment, the composition may comprise various additional phospholipids and glycerides. Further included may be small molecules, such as salts.

In a preferred embodiment of the invention, the composition is, or comprises, lipids obtained by enzymatic conversion of lecithin with phospholipase D. In the enzymatic conversion, phosphatidylserine and phosphatidic acid are preferably obtained as the main products. This can be achieved when the reaction is carried out in the presence of serine. Preferably, the lecithin is vegetable lecithin, such as soybean lecithin, sunflower lecithin or rapeseed lecithin. The lecithin could also be an animal product, such as milk lecithin or egg-yolk lecithin.

Lecithin is a natural source of fatty substances from animal and plant tissues. The main components are phosphoric acid, choline, fatty acids, glycerol, glycolipids, triglycerides and phospholipids. Methods for producing phosphatidylserine and phosphatidic acid from lecithin are known in the art.

EP 1 201 244 A2 discloses a method for producing phosphatidylserine and phosphatidic acid complex by enzymatic conversion with phospholipase D from lecithin. The process is based on a transphosphatidylation and hydrolysis with phospholipase D in the presence of water and an excess of L-serine. Thereby, the choline group of phosphatidylcholine, for example, is substituted by a serine or hydroxyl group, yielding phosphatidylserine or phosphatidic acid in a single reaction. The same principal of head group exchange is found for the different phospholipids in lecithin, such as phosphatidylinositol (PI) and phosphatidylethanolamine (PE). The reaction is carried in a water/oil system. The oily phospholipid fraction can be separated from the aqueous medium. EP 2 151 499 A2 discloses an improved process for simultaneous preparation of a complex of phosphatides, including phosphatidic acid and phosphatidylserine, or salts thereof, by enzymatic processing of lecithin in the presence of phospholipase D.

According to such processes, about 1:1 complexes of phosphatidylserine and phosphatidic acid can be obtained.

Compositions comprising phosphatidylserine and phosphatidic acid in about equal amounts are commercially available from Lonza Ltd., CH, under the trademark MemreePS™ and MemreePlus™.

The composition used in the invention is administered in an effective amount for reducing or preventing sweating. The optimal amount may vary between individuals, depending on factors such as body weight, sex and age.

In a preferred embodiment of the invention, the composition is administered in an amount, such that the daily dosage of component (a) and of component (b), respectively, is at least 50 mg, at least 100 mg or at least 150 mg. Preferably, the daily amount is from 50 to 750 mg, or from 100 to 750 mg, more preferably from 100 to 600 mg.

It was found that daily administration of 400 mg of components (a) and (b), respectively, is even more efficient than administration of 200 mg. Therefore, in preferred embodiments, the daily dose of components (a) and (b), respectively, is at least 250 mg, more preferably at least 300 mg; preferably from 250 to 750 mg, more preferably from 300 to 600 mg, most preferred about 400 mg.

It was found that daily administration of 200 mg of components (a) and (b), respectively, efficiently reduces sweating. This embodiment is advantageous, because pharmacologically active agents generally should be administered in amounts as low as possible. Therefore, in another preferred embodiment, the daily dosage of components (a) and (b), respectively, is from 50 to 300 mg, preferably from 100 to 300 mg, most preferred about 200 mg.

Preferably, the daily dosage of components (a) and (b) together is at least 100 mg or at least 200 mg, more preferably at least 300 mg; preferably from 100 to 1500 mg, more preferably from 200 to 1200 mg.

More preferably, the daily dosage of components (a) and (b) together is at least 500 mg, preferably at least 600 mg; preferably from 500 to 1500 mg, more preferably from 600 to 1200 mg, or about 800 mg. In another preferred embodiment, the daily dosage may be from 100 to 600 mg.

Preferably, the daily dosage of components (a), (b) and (c) together is at least 500 mg, or at least 1000 mg, preferably from 500 to 3000 mg, or from 1000 to 3000 mg, more preferably from 1500 to 2500 mg.

Component (a) may comprise lysophosphatidylserine and/or salts thereof, typically in an amount below 10% (w/w), based on the total of component (a). Preferably, component (a) comprises less than 20% (w/w), more preferably less than 10% (w/w) lysophosphatidylserine and/or salts thereof.

Component (b) may comprise lysophosphatidic acid and/or salts thereof, typically an amount below 10% (w/w), based on the total of component (b). Preferably, component (b) comprises less than 20% (w/w), more preferably less than 10% (w/w) lysophosphatidic acid and/or salts thereof.

In another preferred embodiment of the invention, composition for reducing or preventing hand sweating comprises lipids consisting of
(a) 15 to 40 wt.% phosphatidylserine and lysophosphatidylserine,
(b) 15 to 40 wt.% phosphatidic acid and lysophosphatidic acid and/or salts thereof, and
(c) 70 to 20 wt% additional lipids, especially phospholipids and glycerides,
wherein the total of components (a) to (c) is preferably 100%, the total of lipids in the composition.
wherein components (a) and (b) are obtained at least in part by enzymatic conversion of lecithin with phospholipase D,
wherein the composition is administered in an amount, such that the daily dosage of component (a) and of component (b), respectively, is at least 50 mg, at least 100 mg or at least 250 mg, preferably from 100 mg to 750 mg, or from 250 to 750 mg, or from 100 to 300 mg.

In a preferred embodiment, the composition is administered, preferably orally, 1 to 3 times per day, preferably once or twice per day. Preferably, the compositions are administered with a sufficient quantity of liquid, preferably water. Preferably, the compositions are administered after meals. In a preferred embodiment, the compositions are administered three times per day after meals with a sufficient quantity of liquid.

A highly preferred embodiment of the invention is the use of a composition for reducing or preventing hand sweating, wherein the composition comprises
(a) 75 to 125 mg, preferably about 100 mg, phosphatidylserine and lysophosphatidylserine,
(b) 75 to 125 mg, preferably about 100 mg, phosphatidic acid and lysophosphatidic acid and/or salts thereof, and
(c) 100 to 500 mg, preferably 150 to 350 mg, additional phospholipids and glycerides,
wherein the total of components (a) to (c) is 100%,
wherein the lipids are obtained at least in part by enzymatic conversion of lecithin with phospholipase D,
wherein the composition is administered, preferably orally, 1 to 3 times daily, preferably once or twice daily.

According to the invention, it was found that a daily dosage of about 200 mg component (a) and of about 200 mg component (b) efficiently reduces sweating, especially hand-sweating, especially hand-sweating associated with stress. The effect of a daily dosage of about 400 mg of components (a) and (b), respectively, was even more pronounced.

In a preferred embodiment, the composition is administered at a long-term basis. However, the compositions may also be administered when there is a specific need to prevent sweating, for example when the individual experiences stress or is expecting to experience stress. Preferably, the composition is administered for several days before stress, for example at least 3, at least 7, at least 20 or at least 30 days before. Preferably, the composition is administered over a time period of at least 7 days, or at least 20 or at least 30 days.

In a preferred embodiment, the composition is a food supplement, a food or a beverage. The composition may also be a part thereof. A food supplement (dietary supplement) may consist of the composition, or consist essentially of the composition. Alternatively, the food supplement may comprise the composition and at least one other active ingredient, such as vitamins or minerals. The inventive composition may also be a food or beverage. In such an embodiment, an inventive composition is mixed with a beverage, such as milk, or a food, such as a yogurt.

In a preferred embodiment, the composition is a functional food or part of a functional food. A functional food is a food given an additional function, which is often related to health promotion or disease prevention, by adding one or more new ingredients.

In a preferred embodiment, the composition is for oral administration. However, formulations for other routes of administration are within the scope of the invention. Other routes of administration may, for example, include, rectal, transmucosal, especially transnasal, intestinal or parenteral delivery, including intramuscular, subcutaneous and intramedullary injections as well as intrathecal, direct intraventricular, intravenous, intraperitoneal, intranasal, or intraocular injections.

Compositions for use in the present invention thus may be formulated in conventional manner using one or more physiologically acceptable carriers and excipients. Such additives are not pharmacologically active. They may facilitate processing of the active ingredients into preparations. Proper formulation is dependent upon the route of administration chosen.

Compositions for use in the present invention may be manufactured by processes known in the art, for example by conventional mixing, dissolving, granulating, dragee-making, levigating, emulsifying, encapsulating, entrapping or lyophilizing processes. For oral administration, the composition can be formulated readily by combining the active compounds with acceptable carriers. Such carriers enable the composition to be formulated as tablets, pills, dragees, capsules, liquids, gels, syrups, slurries, suspensions, and the like, for oral ingestion by an individual. Preparations for oral use can be made using a solid excipient, optionally grinding the resulting mixture, and processing the mixture of granules, after adding suitable auxiliaries if desired, to obtain tablets or dragee cores.

The composition may comprise at least one carrier or excipient. The excipient for a powder composition could be an anticaking agent, such as silica. Other suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound dosages.

Compositions which can be used orally include push-fit capsules made of gelatin or vegetable products as well as soft, sealed capsules made of gelatin (e. g. soft or hard) and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added.

In a preferred embodiment of the invention, the composition is a food supplement. The composition may be part of a combination product with other food additives.

The use of the invention may either be a therapeutic use or a non-therapeutic use. The use is therapeutic, if the sweating is pathological. In principle, it is acknowledged that hyperhidrosis can be a pathological condition, which can affect an individual's well-being significantly, and which can be treated by medication.

Another embodiment of the invention is a medicament comprising a composition comprising phosphatidylserine and phosphatidic acid and/or salts thereof for reducing or preventing sweating, especially hand sweating, especially associated with stress. The medicament can be any composition as described herein for the inventive use.

Another embodiment of the invention is a method for reducing or preventing sweating, comprising a step of administering to an individual in need thereof a composition comprising phosphatidylserine and phosphatidic acid and/or salts thereof. The method may be non-therapeutic or therapeutic. In the inventive method, the compositions and medicaments as described herein are applicable.

The inventive use solves the problem underlying the invention. The invention provides an efficient use and composition for reducing or preventing sweating. The composition based on phospholipids is easily available from natural products and applicable for a mild treatment without severe side effects. The composition does not adversely affect vital physiological pathways, in contrast to known hyperhidrosis medicaments, such as anticholinergic agents or botox. In contrast, the composition also relieves psychological stress and improves the well-being of the individuals.

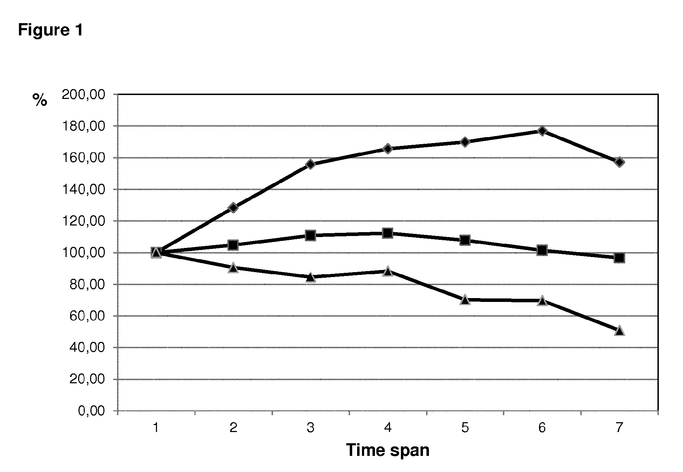

Figure 1: Electrodermal activity results (mean values) of PP population before, during and after the Trier Social Stress test (TSST) as determined in the clinical study according to the examples. The graph shows changes of electrodermal activity in percent versus time points during, before and after TSST. The underlying EDA data is shown in table 1. Time points 1 to 7 correspond to Sitting, Standing (before TSST), Preparation, Interview, Arithmetics (during TSST), Standing and Sitting (after TSST). Initial values at time point 1 (Sitting) were set to 100%. The upper line (diamonds) relates to the placebo group, the middle line (squares) to the 400 mg PAS group, and the lower line (triangles) to the 800 mg PAS group.

### Examples

A clinical study was carried out to determine the effect of administration of phosphatidylserine and phosphatidic acid on sweating of individuals. 75 subjects participated in the study and were separated into three groups.

### A: Compositions

Capsules for 800 mg PAS/day (400 mg PA and 400 mg PS per day) consisted of 100 mg phosphatidylserine (PS) and lysophosphatidylserine, 100 mg phosphatidic acid (PA) and lysophosphatidic acid, 235 mg other phospholipids and glycerides, 5 mg silicon dioxide.

Capsules for 400 mg PAS/day (200 mg PA and 200 mg PS per day) consisted of 50 mg phosphatidylserine (PS) and lysophosphatidylserine, 50 mg phosphatidic acid (PA) and lysophosphatidic acid, 335 mg other phospholipids and glycerides, 5 mg silicon dioxide.

Reference substance / placebo consisted of 435 mg maize starch and 5 mg silicon dioxide.

Placebo and verum groups took the capsules three times per day after meals with a sufficient quantity of liquid (preferably water) for the duration of 42 days (168 capsules in total); two capsules in the morning, one in the afternoon and one in the evening (4 capsules per day).

### B: Subjects

Seventy-five subjects were planned to be included in this study, three subjects dropped out over the course of the study (800 mg PAS group: n= 0; 400 mg PAS group: n = 2; placebo group: n = 1). Subjects were chosen by applying inclusion and exclusion criteria according to common clinical standards. Seventy-five subjects were included in the safety set and 72 in the intent to treat (ITT) population. For analysis of the per protocol (PP) population, 10 subjects of the 800 mg PAS group, 3 subjects of the 400 mg PAS group and 7 subjects of the placebo group were excluded because of poor compliance. One participant was excluded from the 400 mg PAS group because of extreme high EDA values.

### C: Study Procedure

All subjects were asked to take four capsules daily for 42 days: two with breakfast in the morning, one with lunch at midday and one with dinner in the evening. The study was double blinded. Study compliance was controlled by medication event monitoring systems and counting of returned capsules. After 42 days of treatment, the individuals were subjected to a Trier Social Stress Test (TSST) and sweating was monitored as well as physiological parameters. The last treatment took place 90 min. before the TSST at the study site.

### D: Trier Social Stress Test (TRRT)

The TSST lasts approximately 16 min and includes an introduction by the study manager, a preparation phase, an interview for a job in a new company and mental arithmetic. The test duration is unknown to the subject.

Introduction (approximately 2 min.): The study manager (or representative) leads the participant into the TSST room and introduces the TSST setting. Then the subject is asked to imagine that he has applied for a job and is now invited for an interview. The participant will have time to prepare for this interview in the presence of two members of the committee board. The participant is informed that he is video and voice recorded during the stress test. In addition, he learns that both members of the committee are trained in behavioral observation and will document his behavior during the interview as well as the manner and content of his speech. Finally, the study manager asks the subject whether he has any questions regarding the protocol. Questions are answered directly. Thereafter, the subject is asked to start preparing the speech. The study manager leaves the TSST room.

Preparation time (3 min.): The subject is allowed to take notes while preparing the speech.

Interview and assessment of VAS (approximately 6 min.): Now the participant is asked by a member of the committee board to step forward to the microphone and to present an interview for a job in a new company. First the participant gives a free speech, telling why he / she is suited for the job and then is asked questions. The interview part has a duration of five minutes. Afterwards, the participant is asked to fill out the second VAS.

Mental arithmetic task (5 min): The subject is asked to stepwise subtract 17 from 2023 as quickly and correctly as possible. Afterwards, the participant is led back to his room by the study manager.

### E Electrodermal Activity

For each participant, electrodermal activity (EDA) was recorded continuously over a period of approximately 55 min during the TSST visit on the hand back. Assessment started 20 min prior to the TSST, continued throughout the TSST, and ended 20 min after termination of the stress test.

### F: Results

In the PP population, the electrodermal activity (EDA) decreased over time (Table 1, Figure 1). The change over time was statistically significant. The PP population showed a significant Time × Group interaction indicating a decrease in EDA levels in the 800 mg PAS group and an increase in EDA levels in the 400 mg PAS and placebo group (see Table 1). Descriptive statistics are summarized in Table 1. Comparable results were obtained for the ITT population (data not shown).

**Table 1: Electrodermal activity results of PP population during TSST**

| | **Pre-TSST** | | **TSST** | | | **Post-TSST** | |
|---|---|---|---|---|---|---|---|
| | **Sitting** | **Standing** | **Preparation** | **Interview** | **Arithmetic** | **Standing** | **Sitting** |
| **Placebo** | | | | | | | |
| Mean | 2.12 | 2.72 | 3.30 | 3.51 | 3.60 | 3.75 | 3.33 |
| Median | 0.89 | 1.60 | 2.12 | 2.08 | 1.83 | 1.47 | 1.50 |
| SD | 2.74 | 3.51 | 4.08 | 4.40 | 4.91 | 5.05 | 4.29 |
| SE | 0.66 | 0.85 | 0.99 | 1.07 | 1.19 | 1.26 | 1.04 |
| Minimum | 0.50 | 0.50 | 0.50 | 0.50 | 0.50 | 0.51 | 0.51 |
| Maximum | 10.81 | 14.89 | 17.39 | 18.42 | 20.91 | 18.10 | 16.33 |
| N | 17 | 17 | 17 | 17 | 17 | 16 | 17 |
| | | | | | | | |

| 400 mg PAS | | | | | | | |
|---|---|---|---|---|---|---|---|
| Mean | 4.27 | 4.47 | 4.73 | 4.79 | 4.60 | 4.33 | 4.13 |
| Median | 1.60 | 1.83 | 2.16 | 2.39 | 2.23 | 2.23 | 2.19 |
| SD | 10.07 | 9.21 | 8.49 | 8.30 | 7.45 | 6.42 | 6.52 |
| SE | 2.25 | 2.06 | 1.90 | 1.86 | 1.67 | 1.47 | 1.46 |
| Minimum | 0.50 | 0.54 | 0.67 | 0.71 | 0.78 | 0.69 | 0.57 |
| Maximum | 45.95 | 42.52 | 39.62 | 38.76 | 34.45 | 27.96 | 28.88 |
| N | 20 | 20 | 20 | 20 | 20 | 19 | 20 |
| | | | | | | | |

| 800 mg PAS | | | | | | | |
|---|---|---|---|---|---|---|---|
| Mean | 6.77 | 6.13 | 5.72 | 5.97 | 4.75 | 4.71 | 3.43 |
| Median | 2.74 | 2.96 | 3.30 | 3.05 | 3.04 | 2.51 | 2.23 |
| SD | 8.10 | 7.69 | 6.62 | 7.15 | 4.98 | 5.41 | 3.22 |
| SE | 2.09 | 1.99 | 1.71 | 1.85 | 1.33 | 1.40 | 0.83 |
| Minimum | 0.50 | 0.50 | 0.50 | 0.50 | 0.50 | 0.50 | 0.50 |
| Maximum | 26.53 | 23.40 | 24.30 | 23.87 | 16.73 | 17.80 | 11.74 |
| N | 15 | 15 | 15 | 15 | 14 | 15 | 15 |

| | | | | | | | |
|---|---|---|---|---|---|---|---|
| N = sample size, SD = standard deviation, SE = standard error | | | | | | | |

## Claims

1. The use of a composition comprising phosphatidylserine and phosphatidic acid and/or salts thereof for reducing or preventing sweating.

2. The use of claim 1, wherein the use is non-therapeutic.

3. The use of claim 1 or 2, wherein the sweating is hand sweating.

4. The use of at least one of the preceding claims, wherein the sweating is associated with stress.

5. The use of at least one of the preceding claims, wherein the sweating is associated with hyperhidrosis.

6. The use of at least one of the preceding claims, wherein the composition comprises
(a) phosphatidylserine and lysophosphatidylserine, and
(b) phosphatidic acid and lysophosphatidic acid and/or salts thereof.

7. The use of claim 6, wherein the ratio of component (a) to component (b) is between 2:1 and 1:2.

8. The use of at least one of claims 6 and 7, wherein the composition comprises (c) additional phospholipids and glycerides.

9. The use of at least one of the preceding claims, wherein the composition comprises
(a) 5 to 50 wt.% phosphatidylserine and lysophosphatidylserine,
(b) 5 to 50 wt.% phosphatidic acid and lysophosphatidic acid and/or a salts thereof, and
(c) 0 to 90 wt% additional phospholipids and glycerides.

10. The use of at least one of the preceding claims, wherein the composition is, or comprises, lipids obtained by enzymatic conversion of lecithin with phospholipase D.

11. The use of at least one of the preceding claims, wherein the composition is administered in an amount, such that the daily dosage of component (a) and of component (b), respectively, is at least 50 mg, preferably from 100 to 750 mg, more preferably from 250 to 750 mg.

12. The use of at least one of the preceding claims for reducing or preventing hand sweating, wherein the composition comprises lipids consisting of
(a) 15 to 40 wt.% phosphatidylserine and lysophosphatidylserine,
(b) 15 to 40 wt.% phosphatidic acid and lysophosphatidic acid and/or salts thereof, and
(c) 70 to 20 wt% additional lipids, especially phospholipids and glycerides, wherein the total of components (a) to (c) is preferably 100%, the total of lipids in the composition,
wherein components (a) and (b) are obtained at least in part by enzymatic conversion of lecithin with phospholipase D,
wherein the composition is administered in an amount, such that the daily dosage of component (a) and of component (b), respectively, is at least 50 mg, preferably from 100 to 750 mg, more preferably from 250 to 750 mg.

13. The use of at least one of the preceding claims, wherein the composition is a food additive, a beverage or a food, especially a functional food.

14. A medicament comprising a composition comprising phosphatidylserine and phosphatidic acid and/or salts thereof of at least one of the preceding claims for reducing or preventing sweating, preferably hand sweating.

15. A method for reducing or preventing sweating, preferably hand sweating, comprising the step of administering to an individual in need thereof a composition comprising phosphatidylserine and phosphatidic acid and/or salts thereof according to any of the preceding claims.

## Amended claims

### Amended claims in accordance with Rule 137(2) EPC.

1. The non-therapeutic use of a composition comprising phosphatidylserine and phosphatidic acid and/or salts thereof for reducing or preventing sweating.

2. A composition comprising phosphatidylserine and phosphatidic acid and/or salts thereof for use in the treatment of sweating, especially reducing or preventing sweating.

3. The use or composition of claim 1 or 2, wherein the sweating is hand sweating.

4. The use or composition of at least one of the preceding claims, wherein the sweating is associated with stress.

5. The composition of at least one of the preceding claims, wherein the sweating is associated with hyperhidrosis.

6. The use or composition of at least one of the preceding claims, wherein the composition comprises
(a) phosphatidylserine and lysophosphatidylserine, and
(b) phosphatidic acid and lysophosphatidic acid and/or salts thereof.

7. The use or composition of claim 6, wherein the ratio of component (a) to component (b) is between 2:1 and 1:2.

8. The use or composition of at least one of claims 6 and 7, wherein the composition comprises
(c) additional phospholipids and glycerides.

9. The use or composition of at least one of the preceding claims, wherein the composition comprises
(a) 5 to 50 wt.% phosphatidylserine and lysophosphatidylserine,
(b) 5 to 50 wt.% phosphatidic acid and lysophosphatidic acid and/or a salts thereof, and
(c) 0 to 90 wt% additional phospholipids and glycerides.

10. The use or composition of at least one of the preceding claims, wherein the composition is, or comprises, lipids obtained by enzymatic conversion of lecithin with phospholipase D.

11. The use or composition of at least one of the preceding claims, wherein the composition is administered in an amount, such that the daily dosage of component (a) and of component (b), respectively, is at least 50 mg, preferably from 100 to 750 mg, more preferably from 250 to 750 mg.

12. The use or composition of at least one of the preceding claims for reducing or preventing hand sweating, wherein the composition comprises lipids consisting of
(a) 15 to 40 wt.% phosphatidylserine and lysophosphatidylserine,
(b) 15 to 40 wt.% phosphatidic acid and lysophosphatidic acid and/or salts thereof, and
(c) 70 to 20 wt% additional lipids, especially phospholipids and glycerides,
wherein the total of components (a) to (c) is preferably 100%, the total of lipids in the composition,
wherein components (a) and (b) are obtained at least in part by enzymatic conversion of lecithin with phospholipase D,
wherein the composition is administered in an amount, such that the daily dosage of component (a) and of component (b), respectively, is at least 50 mg, preferably from 100 to 750 mg, more preferably from 250 to 750 mg.

13. The use of at least one of the preceding claims, wherein the composition is a food additive, a beverage or a food, especially a functional food.

14. A non-therapeutic method for reducing or preventing sweating, preferably hand sweating, comprising the step of administering to an individual in need thereof a composition comprising phosphatidylserine and phosphatidic acid and/or salts thereof according to any of the preceding claims.
